(19) Europäisches Patentamt
European Patent Office
Office européen des brevets

(11) **EP 2 033 575 B1**

(12) **EUROPÄISCHE PATENTSCHRIFT**

(45) Veröffentlichungstag und Bekanntmachung des Hinweises auf die Patenterteilung:
**28.07.2010 Patentblatt 2010/30**

(21) Anmeldenummer: **08014100.5**

(22) Anmeldetag: **07.08.2008**

(51) Int Cl.:
*A61B 5/04* (2006.01)          *A61B 5/0408* (2006.01)
*A61N 1/04* (2006.01)          *A61B 5/0492* (2006.01)

(54) **Verfahren zur schrittweisen oder kontinuierlichen Herstellung von biomedizinischen Mehrfachelektroden zur Einmalverwendung und aus diesen gebildetes Elektrodensystem**

Method for continuously or step by step manufacturing of biomedical multiple electrodes for single use, and matrix electrode system consisting of same

Procédé de fabrication par étape ou continue d'électrodes biomédicales multiples à usage unique et système d'électrodes ainsi formé

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.09.2007 DE 102007042257**

(43) Veröffentlichungstag der Anmeldung:
**11.03.2009 Patentblatt 2009/11**

(73) Patentinhaber: **Covidien AG**
**8212 Neuhausen am Rheinfall (CH)**

(72) Erfinder:
• **Malkowsky, Andrea**
**38828 Wegeleben (DE)**
• **Anz, Johannes**
**38828 Wegeleben/Adersleben (DE)**
• **Anders, Christoph, Dr.**
**07743 Jena (DE)**
• **Dantlgraber, Gert**
**38820 Halberstadt (DE)**

(74) Vertreter: **Olsson, Carl H.S.**
**Covidien AG**
**Victor von Bruns-Strasse 19**
**8212 Neuhausen am Rheinfall (CH)**

(56) Entgegenhaltungen:
**WO-A-98/02088        DE-A1- 4 336 300**

## Beschreibung

Die Erfindung bezieht sich auf ein Verfahren zur schrittweisen oder kontinuierlichen Herstellung von selbstklebenden, flexiblen biomedizinischen Mehrfachelektroden zur Einmalverwendung bei Menschen oder Tieren, bestehend aus mehreren identischen Elektrodenkörpern, und ein aus diesen gebildetes Elektrodensystem.

Biomedizinische Mehrfachelektroden für unterschiedliche Anwendungen sind bereits seit längerem bekannt.

Aus der DE 43 36 300 A1 ist ein Verfahren zur Herstellung biomedizinischer Solidgelelektroden unter Verwendung einer haftklebstoffbeschichteten Bahn bekannt, wobei die Breite der Bahn so bemessen sein kann, dass eine paarweise Herstellung der Elektroden ermöglicht wird.

Aus der WO 98/02088 A ist ein Verfahren zur Herstellung von biomedizinischen Mehrfachelektroden bekannt, wobei auf einer streifenförmigen Unterlage mehrere abnehmbare Elek-troden hintereinander angeordnet sind.

In der DE 601 14 031 T2 ist eine nichtinvasive, biomedizinische Mehrfachelektrode offenbart, die u.a. zur Identifizierung von peripheren Nerven angewendet wird.

Diese Mehrfachelektrode besteht aus einer Folie aus elektrisch nichtleitendem Material mit einer Sensorelektrodenregion, einer Geräteanschlussregion und einer flexiblen Schaftregion, die die Elektrodensensorregion mechanisch mit der Geräteanschlussregion verbindet. Als Schaltkomplex ist innerhalb der Sensorelektrodenregion eine

Elektrodenanordnung mit mehreren Elektroden bzw. Elektrodenkörpern angeordnet, wobei jede Elektrode über eine Verbindungsleitung mit der Geräteanschlussregion verbunden ist. Die Elektrodenanordnung kann vier in Reihe angeordnete Elektroden bzw. Elektrodenkörper umfassen, wobei die Elektroden jeder Reihe mit den Elektroden der benachbarten Reihe fluchten oder versetzt angeordnet sind. Die Mehrfachelektrode kann z. B. bis zu 64 Elektroden aufweisen.

Die Mehrfachelektroden werden nach der Leiterplattentechnik hergestellt. Die Herstellung ist technologisch aufwendig und kostenintensiv. Elektroden mit einer unterschiedlichen Anzahl an Elektrodenkörpern müssen separat hergestellt werden. Außerdem sind die Mehrfachelektroden jeweils nur für spezifische Anwendungen bestimmt.

Der Erfindung liegt die Aufgabe zugrunde, ein Verfahren zur kontinuierlichen Herstellung von selbstklebenden, flexiblen Mehrfachelektroden zur Einmalverwendung zu schaffen, das eine kostengünstige Herstellung von Mehrfachelektroden mit einer unterschiedlichen Anzahl an Elektrodenkörpern innerhalb einer Fertigungslinie ermöglicht. Ferner soll ein Elektrodensystem bereitgestellt werden, das sich durch verbesserte anwendungstechnische Eigenschaften auszeichnet.

Erfindungsgemäß wird die Aufgabe durch die in den Ansprüchen 1 oder 2 angegebenen verfahrenstechnischen Maßnahmen gelöst. Verfahrenstechnisch vorteilhafte Weiterbildungen sind Gegenstand der Ansprüche 3 bis 10.

Ein geeignetes Elektrodensystem unter Verwendung mehrerer Mehrfachelektroden einer Baureihe ist im Anspruch 11 angegeben. Anspruch 12 bezieht sich auf eine vorteilhafte Ausgestaltungen des Elektrodensystems.

Innerhalb einer Fertigungslinie werden auf dem als endlose Bahnenware in Längsrichtung zugeführten Trägermaterial symmetrisch und identisch aufgebaute Elektrodenkörper gebildet, in einem definierten Rastermaß A, bezogen auf den Abstand in Längsrichtung zwischen den geometrischen Mittelachsen der Elektrodenkörper in 90°-Richtung zur Längsrichtung. Die Bahnenware wird an ihren Längsseiten so beschnitten, dass der Abstand zwischen der Längskante und den zu dieser benachbarten Elektroden gemäß den Ansprüchen 1 oder 2 definiert ist. Anschließend werden die Mehrfachelektroden mit der gewünschten Zahl an Elektrodenkörpern ausgeschnitten oder abgetrennt, wobei die Schnitt- oder Trennlinie jeweils mittig zwischen den Elektrodenkörpern liegt, sodass der Abstand vom Rand zur geometrischen Mittelachse der benachbarten Elektrodenkörper an mindestens drei Seitenkanten der Mehrfachelektroden jeweils die Hälfte (A/2) des Rastermaßes A (Verfahrensweise gemäß Anspruch 1) oder $\sqrt{\dfrac{3}{4}}$ des Rastermaßes A (Verfahrensweise gemäß Anspruch 2) beträgt.

Die Mehrfachelektroden sollten so ausgeschnitten werden, dass diese mindestens zwei geradlinig und parallel verlaufende Seitenkanten aufweisen.

Es besteht auch die Möglichkeit, dass das Ausschneiden bzw. Abtrennen der Mehrfachelektroden erst unmittelbar vor der Applikation der jeweiligen Mehrfachelektroden vorgenommen wird. In diesem Fall werden in eine flächenförmige Einheit mit einer bestimmten Anzahl an Elektroden mittig zwischen den Elektrodenkörpern vorbereitete Trennschnitte eingebracht, sodass die Mehrfachelektroden mit der gewünschten Anzahl an Elektrodenkörpern manuell abgetrennt werden können.

Die Größe des Rastermaßes A ist unter anderem von der Größe der Hydrogelkörper und dem späteren Einsatzgebiet abhängig und liegt in einem Bereich von 9 bis 120 mm, vorzugsweise 14 bis 33 mm.

Der gelfreie Abstand zwischen den einzelnen Hydrogelkörpern beträgt in Abhängigkeit von der Größe der Gelkörper und dem Rastermaß A mindestens 1,5 mm, vorzugsweise bis 25 mm. Die gebildeten Hydrogelkörper besitzen eine symmetrische Form, vorzugsweise ist diese kreisrund oder rechteckig.

Die Größe der Gelkörper beträgt 7 bis 25 mm, vorzugsweise von 12 bis 21 mm, wobei dieses Maß sich auf den Durchmesser oder die Länge einer Kante bezieht.

Vorzugsweise werden die Elektrodenkörper in einer oder mehreren parallel zueinander verlaufenden Reihen, die in Längsrichtung der Bahnenware verlaufen, angeordnet. Es besteht auch die Möglichkeit, wie im Anspruch 2 angegeben, die Elektrodenkörper-Reihen versetzt zueinander anzuordnen, derart, dass jeweils die geometrischen Mittelachsen dreier benachbarter Elektrodenkörper ein gleichseitiges Dreieck bilden. In diesem Fall entspricht die Anordnung der Elektrodenkörper einer parallelogrammartigen Struktur.

Zum Abziehen der der hautseitigen Abdeckung der Mehrfachelektrode wird diese mit einer überstehenden Abgrifflasche ausgerüstet, die vorzugsweise an einer der Schmalseiten angeordnet wird. Die Abgrifflasche kann auch in die Form integriert oder seitlich angebracht werden.

Zur Erleichterung der Applikation von mehreren Mahrfachelektroden unmittelbar nebeneinander wird an mindestens einer der Längskanten der Mehrfachelektroden das Abstandsmaß zwischen zwei benachbarten Elektrodenkörpern durch eine optisch wahrnehmbare Markierung an der von der Haut abgewandten Seite gekennzeichnet.

Zur Bildung der Hydrogelkörper wird ein ionisch leitfähiges Gebilde auf Basis von Solid- oder Liquidgel eingesetzt.

Der prinzipielle Aufbau der Mehrfachelektroden entspricht herkömmlichen Ausführungen, wie sie von Einzelelektroden her bekannt sind.

Das Trägermaterial kann aus einem flexiblen Material auf Basis eines Polymerschaums, einer kompakten Polymerfolie, eines Vlieses oder Gewebes oder beliebigen Kombinationen daraus bestehen. Das flächige Trägermaterial ist mindestens hautseitig mit einem hautverträglichen Permanentklebstoff ausgerüstet.

Die klebende, hautzugewandte Seite der Mehrfachelektrode, bestehend aus Hydrogel und möglicherweise auch dem adhäsiv ausgerüsteten Trägermaterial, wird mit einem wiederablösbaren Flächenmaterial, wie einer silikonisierten Polymerfolie, bis zur unmittelbaren Applikation abgedeckt. Das an sich flüsslgkoltsdurchlässlge, flächige Trägermaterial kann mindestens hautseitig flüssigkeitsundurrhlässig ausgerüstet sein. Der jeweilige Sensor besteht aus einem gelverträglichen, mindestens oberflächlich elektrisch leitenden Körper, vorzugsweise mit einer Silber- Silberchlorid- Oberfläche.

Die elektrische Verbindung zwischen Sensor und dem peripheren Gerät erfolgt mittels üblicher Leitungen, die fest oder über entsprechende Adapter lösbar mit dem Sensor verbunden sind.

Zur elektrischen Verbindung können übliche Druckknöpfe aus Metall oder Kunststoff eingesetzt werden, analog wie bei EKG-Elektroden. Die Sensor-Druckknoptverbindung kann auch röntgentransluzent ausgeführt sein. Eine an den Sensor angeschlagene Litze kann geräteseitig mit einem spezifischen Adapter ausgerüstet sein.

Die vorgeschlagene Verfahrensweise ermöglicht eine besonders kostengünstige Herstellung von Mehrfachelektroden mit einer unterschiedlichen Anzahl an Elektrodenkörpern innerhalb einer Fertigungslinie. Die Mehrfachelektroden können für unterschiedliche klinische Applikationen der Zu- oder Ableitung elektrischer Signale über die Haut, wie z.B. zur Muskelstimutation, zur Erfassung des OEMG

(Oberflächenelektromyographle), zur Messung des EKG (Elektrokardiogramm), zur EIT (Elektroimpedanztomographie) usw. eingesetzt werden, gegebenenfalls auch In Kombination mit Einzelelektroden.

Die Konfiguration der Symmetrie der vorgeschlagenen Mehrfachelektroden mit dem Rastermaß A/2 (Verfahrensweise gemäß Anspruch 1) oder $\frac{\sqrt{3}}{4}$ (Verfahrensweise gemäß Anspruch 2) der hergestellten Elektroden als seitlicher Abstand ermöglicht die Anwendung sogenannter Elektrodenraster: dabei werden eine Vielzahl (z.B.16 oder auch 32) von Elektroden In einem symmetrischen Abstandsverhältnis zueinander benötigt. Dadurch wird eine quasi flächendeckende Untersuchung von Muskeln ermöglicht. Bei der monopolaren Verschaltung werden die jeweiligen Messelektroden durch Interne Verschaltung gegen eine Referenzelektrode verschaltet. Bei der bipolaren Verschaltung werden jeweilige Spannungsdifferenzen von Elektrodenpaarungen ausgewertet.

Ein weiterer wesentlicher Vorteil besteht in der freien Kombination von mehreren Mehrfachelektroden mit einer gleichen oder unterschiedlichen Anzahl an Elektrodenkörpern zu einem Elektrodensystem.

Die erfindungsgemäß hergestellten Mehrfachelektroden einer Baureihe können mit mindestens einer ihrer Längs- oder Querkanten vollständig oder teilweise aneinander stoßend zu unterschiedlich großen und geometrisch unterschiedlichen Applikationsflächen kombiniert werden, wobei alle Elektrodenkörper der Applikationsfläche zueinander, bezogen auf ihre geometrischen Mittelachsen, im gleichen Rastermaß angeordnet sind. Der Abstand der äußeren Elektrodenkörper zu der äußeren Begrenzung der Applikationsfläche beträgt einheitlich A/2 oder $\frac{\sqrt{3}}{4}$ des Rastermaßes A bei versetzt zueinander angeordneten Elektrodenkörper-Reihen.

Dabei ist auch eine versetzte Anordnung aneinander gereihter Mehrfachelektroden möglich.

Die Aneinanderreihung der Mehrfachelektroden kann unmittelbar während der Applikation erfolgen. Über entsprechende Markierungen an der Oberseite der Elektroden wird eine genaue Ausrichtung der Mehrfachelektroden gewährleistet, sodass alle Elektrodenkörper nach der Applikation symmetrisch im Rastermaß A angeordnet sind. Dies ist von wesentlicher Bedeutung, da nur bei genau definierten und identischen Abständen zwischen den einzelnen Elektrodenkörpern entsprechende klinische Messmethoden fehlerfrei arbeiten, wie dies insbesondere zur räumlichen Filterung von OEMG-Signalen aus monopolaren Meßanordnungen erforderlich ist.

Die Sensoren aller Elektrodenkörper einer Applikatlonsfläche sind über separate Leitungen an das zugehörige Peripheriegerät angekoppelt.

Durch die Anwendung räumlicher Filter können biologisch bedingte Einflüsse, vor allem die unwillkürliche Aktivität benachbarter (seitlich oder in unterschiedlicher Tiefe unter der Haut befindlicher) Muskeln verringert werden. Die erfindungsgemäß hergestellten Mehdachelektroden sind aufgrund der Anordnung der Elektrodenkörper in einer Matrix (Rastermaß A) und Randabstand (A/2 oder $\sqrt{\frac{3}{4}}$ von A) hierfür besonders gut geeignet. Um den jeweiligen anatomischen und messtechnischen Bedingungen Rechnung tragen zu können, ist es sinnvoll, verschiedene Filter zu verwenden. Dies kann durch die Applikation mehrerer aneinander gereihter Mohrfachoioktroden erreicht werden.

Die erfindungsgomäßen Mehrfachelektroden ermöglichen, dass bei gleich bleibender Symmetrie- und Elektrodenabstandssituation auf dem Muskel der interessierende Bereich immer vollständig erfasst werden kann und somit gut vergleichbare Messergebnisse erhalten werden.

Durch geometrisch ungeordnete Aneinanderreihungen verschiedener Mehrfachelektroden können mit weniger Aufwand für das klinische Personal Anordnungen appliziert werden, wie sie für EKG-Messungen benötigt werden.

Die Erfindung soll nachstehend an einigen Beispielen erläutert werden, in der zugehörigen Zeichnung zeigen:

Fig. 1 eine Mehrfachelektrode mit drei Elektrodenkörpern als Draufsicht,

Fig. 2 eine Mehrfachelektrode mit sechs Elektrodenkörpern als Draufsicht,

Fig. 3 eine Mehrfachelektrode mit zwölf Elektrodenkörpern als Draufsicht,

Fig. 4 eine erste Ausführung zur Bildung einer Applikatlonsfläche mit den Mehrfachelektroden gemäß den Figuren Fig. 1 bis 3,

Fig. 5 eine zweite Ausführung zur Bildung einer Applikationsfläche mit den Mehrfachelektroden gemäß den Figuren Fig. 1 bis 3,

Fig. 6 eine Mehrfachelektrode mit acht Elektrodenkörpern als Draufsicht und

Fig. 7 eine Mehrfachelektrode mit versetzt zueinander angeordneten Elektrodenkörpern als Draufsicht.

Die Herstellung der gezeigten Mehrfachelektroden mit einem kreisrunden Gelkörper erfolgt entweder kontinuierlich oder schrittweise in einem Fertigungsautomaten als Herstellungslinie. Diesem wird als Trägermaterial eine mit hautverträglichem Permanentklebstoff beschichtete Polyethylen-Schaumbahn 1 als Endloxbahn zugeführt, die eine Breite von 90 mm besitzt und in ihrer Breite zur Anordnung von drei parallelen Reihen an Elektrodenkörpern bestimmt ist. Der zugehörige Originalliner wird beidseitig, nahe der Kanten eingeschnitten. Mittels einer elektronisch gesteuerten Schneidvorrichtung werden die zur späteren Aufnahme des Gels erforderlichen kreisrunden Kavitäten 2 ausgestanzt, die einen Durchmesser von 16 mm besitzen. Der Stanzvorgang erfolgt so, dass die einzelnen, symmetrischen Kavitäten mit gleichgroßem Durchmesser in drei parallel angeordneten Reihen gebildet werden, in einem definierten Rastermaß A von 25 mm, bezogen auf den Abstand In Längsrichtung zwischen den geometrischen Mittelachsen x der Kavitäten in 90°-Richtung zur Längsrichtung. Der außerhalb der Fixierklemmen zum Halt des Schaums befindliche Liner wird abgehoben, während die beiden Randbereiche des Originalliners infolge des o.g. Einschnitts auf der Schaumstoffbahn verbleiben. Die Kavitätsöffnungen 2 werden mittels eines Labels 3 verschlossen. Zur elektrischen Signalübertragung werden oberseitig ein Metalldruckknopf 4 und gel- bzw. hautseitig ein mit einer Silber-Silberchlorid-Schicht überzogener Sensor kraftschlüssig miteinander verbunden, wobei zwischen beide Teile das Label 3 eingeklemmt wird. Anschließend wird in die von unten abgedeckten Kavitäten 2 die Reaktionsmischung zur Bildung der Gelkörper 5 eingebracht. In einer nachfolgenden UV-Selichtungsstrecke wird die Reaktionsmischung durch Polymerisation verfestigt. Alle klebenden, hautseitigen Flächen werden mittels eines wieder ablösbaren Liners bis zur unmittelbaren Applikation auf der Haut schützend abgedeckt Die Gelkörper 5 sind, geometrisch getrennt und elektrisch voneinander isoliert, in drei Reihen angeordnet. In einem weiteren Schneldvorgang wird die Schaumstoffbahn an ihren Längsseiten so beschnitten, dass der Abstand zwischen der Längskante und den zu dieser benachbarten Elektrodenkörpern die Hälfte des Rastermaßes A beträgt.

Abschließend werden die Mehrfachelektroden ausgeschnitten, entsprechend der gewünschten Anzahl an Elektrodenkörpern, wobei der Schnitt jeweils mittig und geradlinig zwischen den Elektrodenkörpern erfolgt. Die Mehrfachelektroden einer Baureihe besitzen eine gleichgroße oder unterschiedliche Anzahl an identischen Elektrodenkörpern 6, die zueinander alle im gleichen Rastermaß A angeordnet sind. Die einzelnen Mehrfachelektroden 7 weisen auch an mindesten drei Seiten jeweils vom Rand bis zur geometrischen Mittelachse der benachbarten Elektrodenkörper einen konstanten Abstand B auf, der die Hälfte des Rastermaßes A (A/2) beträgt.

An einer der Mehrfachelektroden ist eine Grifflasche 8 angebracht Diese erleichtert sowohl das Trennen von Liner und der eigentlichen Mehrfachelektrode vor der klinischen Applikation als auch das Wiederentfernen der Mehrfachelektrode von der Haut mit Beenden der Applikation. Die hautzugewandte Unterseite der Grifflasche 8 ist mittels eines nichtklebenden Flächengebildes, z.B. Teilen des den Schaum ursprünglich abdeckenden Originalliners, nichtklebend abgedeckt.

Der angebrachte Metaildruckknopf 4 zur Signalübertragung befindet sich jeweils in der Mitte des kreisrunden Gelkörpers 5. Über eine entsprechende elektrische Leitung mit Druckknopfanschluss erfolgt dann die Verbindung zum zugehörigen peripheren Gerät.

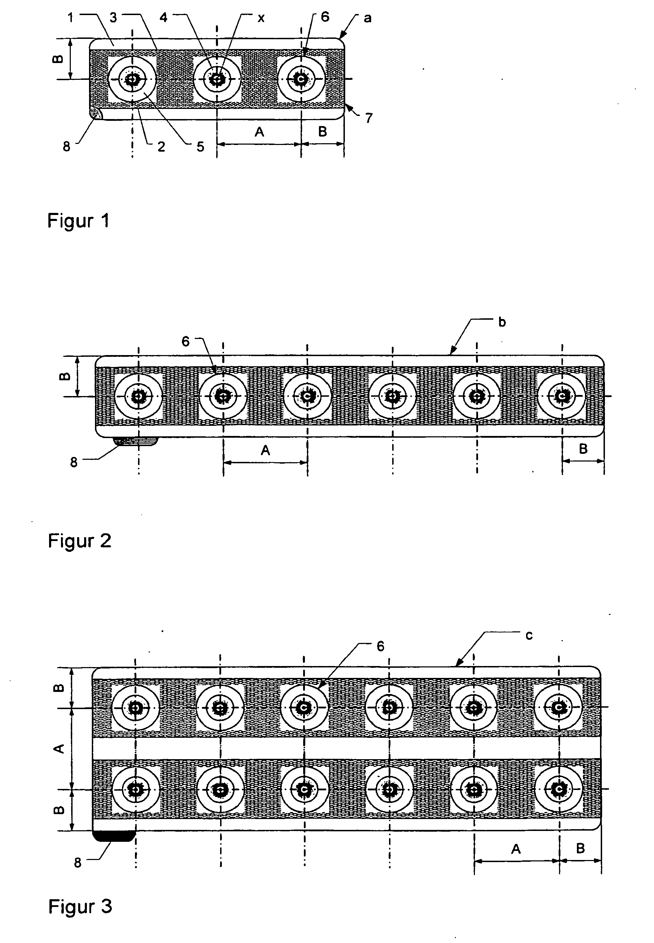

In den Figuren 1 bis 3 sind drei Mehrfachelektroden a, b, c einer Baureihe mit einer unterschiedlichen Anzahl an identischen Elektrodenkörpern gezeigt, die nach vorgenannter Verfahrensweise hergestellt wurden.

Die Mehrfachelektrode a ist mit 3, die Mehrfachelektrode b mit 6 und die Mehrfachefektrode c mit 12 Elektrodenkörpern 6 ausgerüstet. Die Grifflaschen 8 der Mehrfachelektroden a, b, c sind Jeweils an unterschiedlichen Seiten angebracht.

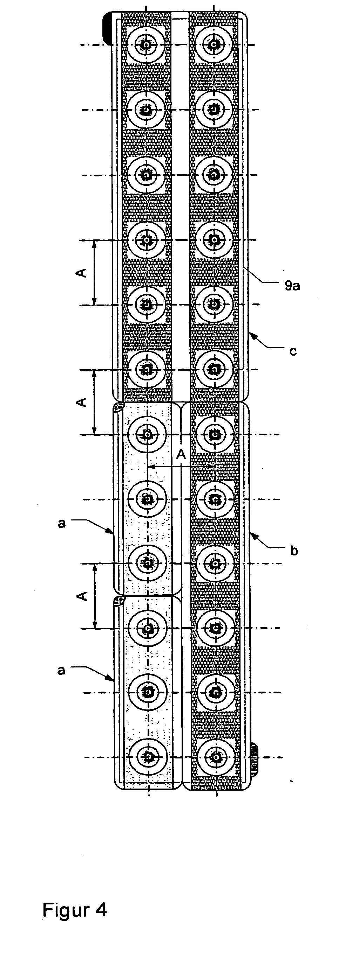

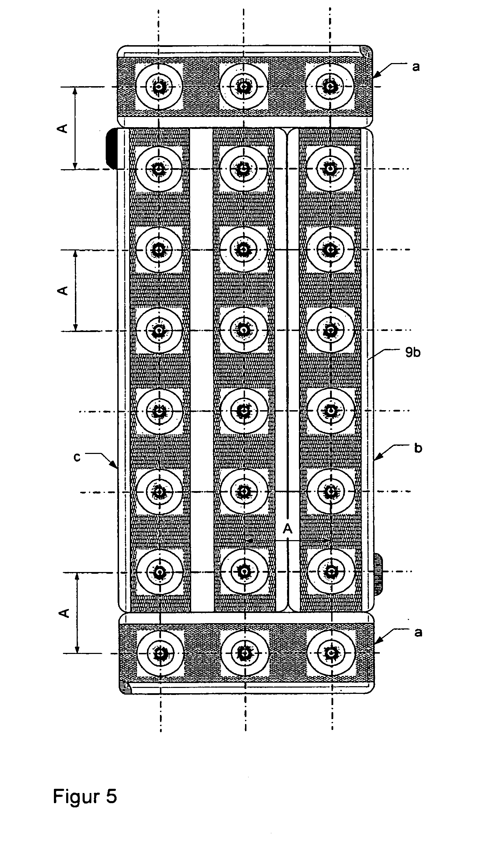

In den Figuren 4 und 5 ist an zwei Ausführungsvarianten gezeigt, wie die Mehrfachelektroden a, b, c zur Bildung unterschiedlich großer und geometrisch unterschiedlicher Applikationsflächen 9a und 9b auf der Haut des Patienten aneinandergereiht werden können. In Fig. 4 sind die Mehrfachelektrode a und b jeweils an den Seitenkanten der Mehrfaahelektrode c "Stoß an Stoß" angeordnet.

In Fig. 5 ergeben die Mehrfacheiektroden a, b, c eine dreireihige Matrix.

Alle Elektrodenkörper 6 der Mehrfachelektroden a, b, c der Applikationsflächen 9a und 9b sind zueinander nach erfolgter Applikation im gleichen Rastermaß A angeordnet.

Somit ist es möglich, mittels mehrerer Mehrfachelektroden einer Baureihe für die jeweilige medizinische Anwendung erforderliche Applikationsflächen zu bilden. Eine

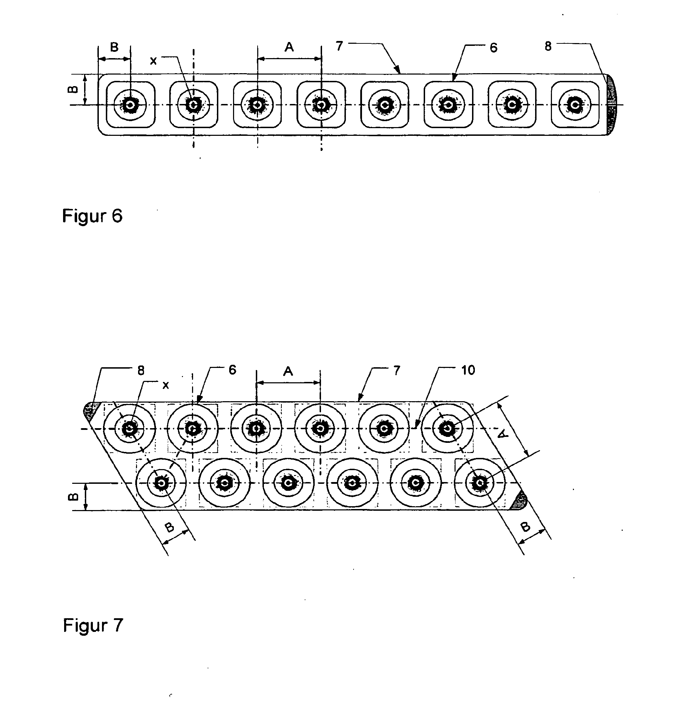

Sonderanfertigung einer ansonsten hierfür erforderlichen Mehrfachelektrode ist somit nicht mehr erforderlich. Für den Anwender ergeben sich somit erhebliche Kosteneinsparungen. In Figur 6 Ist eine Mehrfachelektrode 7 dargestellt, die mit in einer Reihe angeordneten acht Elektrodenkörpern 6 ausgerüstet ist. Die einzelnen Elektrodenkörper 6 besitzen eine quadratische Querschnittsfläche mit abgerundeten Ecken und sind zueinander im Rastermaß A, bezogen auf den Abstand In Längsrichtung zwischen den geometrischen Mittelachsen x der Elektrodankörper in 90°-Richtung zur Längsrichtung angeordnet. Die Grifflasche 8 ist an einer der Schmalseiten angeordnet. Die Abstände von der Mittelachse x der Elektrodenkörper 6 zu den weiteren drei Seitenkanten betragen jeweils A/2. Die Herstellung dieser Mehrfachelektroden erfolgt in ähnlicher Weise wie vorstehend erläutert. Es besteht die Möglichkeit, diese Mehrfachelektroden aus einer einreihigen oder mehrreihigen Bahnenware auszuschneiden.

In Figur 7 ist eine Mehrfachelektrode 7 mit 12 Elektrodenkörpern 6 gezeigt, wobei jeweils 6 Elektrodenkörper 6 eine Reihe 10 bilden. Die beiden parallel verlaufenden-Reihen 10 sind versetzt zueinander angeordnet, derart, dass die jeweiligen Mittelachsen x dreier benachbarter Elektrodenkörper 6 ein gleichseitiges Dreieck bilden. Die einzelnen Elektrodenkörper 6 besitzen zueinander den gleichen Abstand im Rastermaß A, bezogen auf die Mittelachse x. Von diesen aus beträgt der Abstand B zu der benachbarten Seitenkante $\frac{\sqrt{3}}{4} A$ . Die Mehrfachelektrode hat in ihrer Grundfläche die Form eines Parallelogramms. An jeweils zwei gegenüberliegenden Eckbereichen ist eine Grifflasche 8 angeordnet. Mehrere dieser Mehrfachelektroden können sowohl an ihren Längs- als auch Schmalseiten zu unterschiedlich großen Applikationsflächen aneinandergereiht werden, ohne Veränderung des Rastermaßes A der Elektrodenkörper 6.

Bezugszeichen

| 1 | Polyethylen-Schaumbahn (Trägermaterial) |
| 2 | Kavität |
| 3 | Label |
| 4 | Metaildruckknopf |
| 5 | Reaktionsmischung/Gelkörper |
| 6 | Elektrodenkörper |
| 7 | Mehrfachelektrode |
| 8 | Grifflasche |
| 9a | Applikationsfläche |
| 9b | Applikationsfläche |
| 10 | Reihe |
| A | Rastermaß |
| B | Rastermaß A/2 bzw. $\frac{\sqrt{3}}{4} A$ |
| a | Mehrfachelektrode einer Baureihe |
| b | Mehrfachelektrode einer Baureihe |
| c | Mehrfachelektrode einer Baureihe |
| x | Mittelachse |

**Patentansprüche**

1. Verfahren zur schrittweisen oder kontinuierlichen Herstellung von selbstkiebenden, flexiblen Mehrtachelektroden (7, a, b, c) zur Einmalverwendung bei Menschen oder Tieren, bestehend aus mehreren identischen Elektrodenkörpern (6), die jeweils aus einer Hydrogel-Sensor-Einheit bestehen, zur nichtinvasiven Übertragung elektrischer Signale von der Haut zu einem elektronischen Gerät oder zur Übertragung elektrischer Signale zur Haut, wobei zur Bildung der voneinander isolierten Elektrodenkörper (6) in ein flexibles, selbstklebendes Trägermaterial (1) Aufnahmen für eine Mischung zur Bildung der Hydrogelkörper (5) eingebracht werden oder die Mischung an bestimmten Stellen auf das Trägermaterial (1) aufgebracht wird und an der von der Haut abgewandten Seite ein Anschluss an die elektrisch leitenden Sensoren (4) zur Ankopplung an die Verbindungsleitungen des elektronischen Gerätes angebracht wird, und auf dem als endlose Bahnenware in Längsrichtung zugeführten Trägermaterial (1) symmetrisch und identisch aufgebaute Elektrodenkörper (6) gebildet werden, in einem definierten Rastermaß (A), bezogen auf den Abstand in Längsrichtung zwischen den geometrischen Mittelachsen (x) der Elektrodenkörper In 90°-Richtung zur Längsrichtung, in einer oder mehreren parallel zueinander angeordneten Reihen (10), die in Längsrichtung der Bahnenware verlaufen, **dadurch gekennzeichnet, dass** die Bahnenware an ihren Längsseiten so beschnitten wird, dass der Abstand (B) zwischen der Längskante und den geometrischen Mittelachsen (x) der zu dieser Längskante benachbarten Elektrodenkörper (6) die Hälfte des Rastermaßes (A) beträgt, und die Mehrfachelektroden (7, a, b, c) mit der gewünschten Zahl an Elektrodenkörpern (6) ausgeschnitten oder abgetrennt werden, wobei die Schnitt- oder Trennlinie jeweils mittig zwischen den Elektrodenkörpern (6) liegt, sodass der Abstand (B) vom Rand zur geometrischen Mittelachse (x) der benachbarten Elektrodenkörper (6) an mindestens drei Seitenkanten der Mehrfachelektroden (7, a, b, c) jeweils die Hälfte des Rastermaßes (A) beträgt.

2. Verfahren zur schrittweisen oder kontinuierlichen Herstellung von selbstklebenden, flexiblen Mehrfachelektroden (7) zur Einmalverwendung bei Menschen oder Tieren, bestehend aus mehreren identischen Elektrodenkörpern (6), die jeweils aus einer Hydroget-Sensor-Einheit bestehen, zur nichtinvasiven Übertragung elektrischer Signale von der Haut zu einem elektronischen Gerät oder zur Übertragung elektrischer Signale zur Haut, wobei zur Bildung der voneinander isolierten Elektrodenkörper (6) in ein flexibles, selbstklebendes Trägermaterial (1) Aufnahmen für eine Mischung zur Bildung der Hydrogelkörper (5) eingebracht werden oder die Mischung an bestimmten Stellen auf das Trägermaterial (1) aufgebracht wird und an der von der Haut abgewandten Seite ein Anschluss an die elektrisch leitenden Sensoren (4) zur Ankopplung an die Verbindungsieltungen des elektronischen Gerätes angebracht wird, und auf dem als endlose Bahnenware in Längsrichtung zugeführten Trägermaterial (1) symmetrisch und identisch aufgebaute Elektrodenkörper (6) gebildet werden, in einem definierten Rastermaß (A), bezogen auf den Abstand in Längsrichtung zwischen den geometrischen Mittelachsen (x) der Elektrodenkörper in 90°-Richtung zur Längsrichtung, in einer oder mehreren parallel zueinander angeordneten Reihen (10), die in Längsrichtung der Bahnenware verlaufen, **dadurch gekennzeichnet, dass** die Elektrodenkörper-Reihen (10) versetzt zueinander angeordnet werden, derart, dass jeweils die geometrischen Mittelachsen (x) dreier benachbarter Elektrodenkörper (6) ein gleichseitiges Dreieck bilden, die Bahnenware an ihren Längsseiten so beschnitten wird, dass der Abstand (B) zwischen der Längskante und den geometrischen Mittelachsen (x) der zu diesor Längskante benachbarten Elektrodenkörper (6) $\dfrac{\sqrt{3}}{4}$ des Rastermaßes (A) beträgt, und die Mehrfachelektroden (7) mit der gewünschten Zahl an Elektrodenkörpern (6) ausgeschnitten oder abgetrennt werden, wobei die Schnitt- oder Trennlinie jeweils mittig zwischen den Elektrodenkörper (6) liegt, sodass der Abstand (B) vom Rand zur geometrischen Mittelachse (x) der benachbarten Elektrodenkörper (6) an mindestens drei Seitenkanten der Mehrfachelektroden (7) jeweils $\dfrac{\sqrt{3}}{4}$ des Rastermaßes (A) beträgt.

3. Verfahren nach einem der Ansprüche 1 oder 2, **dadurch gekennzeichnet, dass** die Größe des Rastermaßes A In Abhängigkeit von der Größe der Hydrogelkörper (5) in einem Bereich von 9 bis 120 mm liegt.

4. Verfahren nach einem der Ansprüche 1 bis 3, **dadurch gekennzeichnet, dass** der gelfreie Abstand zwischen den einzelnen Hydrogelkörpern (5) in Abhängigkeit von der Größe der Gelkörper mindestens 1,5 mm beträgt

5. Verfahren nach einem der Ansprüche 1 bis 4, **dadurch gekennzeichnet, dass** die Hydrogelkörper (5) einen Durchmesser oder eine Kantenlänge von 7 bis 25 mm besitzen.

6. Verfahren nach einem der Ansprüche 1 bis 5, **dadurch gekennzeichnet, dass** die Mehrfachelektroden (7, a, b, c) so ausgeschnitten werden, dass diese mindestens zwei geradlinig und parallel verlaufende Seitenkanten aufweisen.

7. Verfahren nach einem der Ansprüche 1 bis 6, **dadurch gekennzeichnet, dass** an einer der Schmalseiten der Mehrfachelektroden (7, a, b, c) eine überstehende Grifflasche (8) zum Abziehen der hautseitigen Abdekkung angeordnet wird.

8. Verfahren nach einem der Ansprüche 1 bis 7, **da-**

**durch gekennzeichnet, dass** an mindestens einer der Längskanten der Mehrfachelektroden (7, a, b, c) das Abstandsmaß (A/2, B) zwischen zwei benachbarten Elektrodenkörpern (6) durch eine optisch wahrnehmbare Markierung an der von der Haut abgewandten Seite **gekennzeichnet** wird.

9. Verfahren nach einem der Ansprüche 1 bis 8, **dadurch gekennzeichnet, dass** zur Bildung der Hydrogelkörper (5) ein ionisch leitfähiges Gebilde auf Basis von Solid- oder Liquidgel eingesetzt wird.

10. Verfahren nach einem der Ansprüche 1 bis 9, **dadurch gekennzeichnet, dass** mittig zwischen den Elektrodenkörpern (6), vorbereitete Trennschnitte eingebracht werden, sodass die Mehrfachelektroden (7, a, b, c) mit der gewünschten Anzahl an Elektrodenkörpern (6) manuell abtrennbar sind.

11. Elektrodensystem, bestehend aus mehreren Mehrfachelektroden einer Baureihe, hergestellt nach einem der vorhergehenden Ansprüche 1 bis 10, **dadurch gekennzeichnet, dass** mehrere Mehrfachelektroden (7, a, b, c) mit gleicher oder unterschiedlicher Anzahl an Elektrodenkörpern (6) mit mindestens einer ihrer Längs- oder Querkanten vollständig oder teilweise aneinander stoßend zu unterschiedlich großen und geometrisch unterschiedlichen Applikatlonsflächen (9a, 9b) zusammengesetzt sind, wobei alle Elektrodenkörper (6) der Applikatiohsfläche (9a, 9b) zueinander, bezogen auf ihre geometrischen Mittelachsen (x), im gleichen Rastermaß (A) angeordnet sind.

12. Elektrodensystem nach Anspruch 11, **dadurch gekennzeichnet, dass** die Sensoren aller Elektrodenkörper (6) der Äpplikationsfläche (9a, 9b) über separate Leitungen an ein Peripheriegerät ankoppelbar sind.

**Claims**

1. A method for progressively or continuously manufacturing self-adhesive flexible multiple electrodes (7, a, b, c) for single use in humans or animals, consisting of several identical electrode bodies (6), respectively consisting of a hydrogel/sensor unit, for non-invasively transmitting electrical signals from the skin to an electronic device, or for transmitting electrical signals to the skin, wherein for forming the mutually isolate electrode bodies (6), sockets for a mixture for forming the hydrogel bodies (5) are made in a flexible self-adhesive backing material (1), or the mixture is applied to the backing material (1) at certain positions, and on the side turned away from the skin, a connector is fitted to the electrically conducting sensors (4) for coupling with the connecting lines of the electronic device, and on the backing material (1), fed in the longitudinal direction as endless sheet material, electrode bodies (6) of symmetrical and identical construction are formed in a defined modular size (A), with respect to the spacing in the longitudinal direction between the geometric centerlines (x) of the electrode bodies in the direction of 90° to the longitudinal direction, in one or more rows (10) arranged parallel to one another, extending in the longitudinal direction of the sheet material, **characterized in that** the sheet material is trimmed at the longitudinal sides thereof so that the spacing (B) between the longitudinal edge and the geometric centerlines (x) of the electrode bodies (6) adjacent to this longitudinal edge is half the modular size (A), and the multiple electrodes (7, a, b, c) are cut out or divided with the desired number of electrode bodies (6), wherein the cutting or dividing line is located respectively in the middle between the electrode bodies (5), so that the spacing (B) from the edge to the geometric centerline (x) of the adjacent electrode bodies (6) at least at three longitudinal edges of the multiple electrodes (7, a, b, c) is respectively half the modular size (A).

2. A method for progressively or continuously manufacturing self-adhesive flexible multiple electrodes (7) for single use in humans or animals, consisting of several identical electrode bodies (6), respectively consisting of a hydrogel/sensor unit, for non-invasively transmitting electrical signals from the skin to an electronic device, or for transmitting electrical signals to the skin, wherein for forming the mutually isolated electrode bodies (6), sockets for a mixture for forming the hydrogel bodies (5) are made in a flexible self-adhesive backing material (1), or the mixture is applied to the backing material (1) at certain positions, and on the side turned away from the skin, a connector is fitted to the electrically conducting sensors (4) for coupling with the connecting lines of the electronic device, and on the backing material (1), fed in the longitudinal direction as endless sheet material, electrode bodies (6) of symmetrical and identical construction are formed in a defined modular size (A), with respect to the spacing in the longitudinal direction between the geometric centerlines (x) of the electrode bodies in the direction of 90° to the longitudinal direction, in one or more rows (10) arranged in parallel to one another, extending in the longitudinal direction of the sheet material, **characterized in that** the rows of electrode bodies (10) are arranged with an offset to one another, so that respectively the geometric centerlines (x) of three adjacent electrode bodies (6) will form an equilateral triangle, the sheet material is trimmed at the longitudinal sides thereof so that the spacing (B) between the longitudinal edge and the geometric centerlines (x) of the electrode bodies (6) adjacent to this longitudinal edge is $\frac{\sqrt{3}}{4}$ the modular size (A), and the multiple electrodes (7) are cut out or divided with the desired number of electrode bodies (6), wherein the cutting or dividing line is respectively located in the middle between the electrode bodies (6), so that the spacing (B) from the edge to the geometric centerline (x) of the adjacent electrode bodies (6) at least at three longitudinal edges of the multiple electrodes (7) is respectively $\frac{\sqrt{3}}{4}$ the modular size (A).

3. The method according to any of claims 1 or 2, **characterized in that** the size of the modular size A lies in a range from 9 to 120 mm depending on the size of the hydrogel bodies (5).

4. The method according to any of claims 1 to 3, **characterized in that** the gel-free spacing between the individual hydrogel bodies (5) is at least 1.5 mm depending on the size of the gel bodies.

5. The method according to any of claims 1 to 4, **characterized in that** the hydrogel bodies (5) have a diameter or edge length from 7 to 25 mm.

6. The method according to any of claims 1 to 5, **characterized in that** the multiple electrodes (7, a, b, c) are cut out such that they have at least two longitudinal edges extending linearly and in parallel.

7. The method according to any of claims 1 to 6, **characterized in that** on one of the small faces of the multiple electrodes (7, a, b, c) a protruding gripping tab (8) is arranged for peeling off the skin-facing coating.

8. The method according to any of claims 1 to 7, **characterized in that** on at least one of the longitudinal edges of the multiple electrodes (7, a, b, c), the clearance (A/2, B) between two adjacent electrode bodies (6) is identified by an optically visible mark on the side turned away from the skin.

9. The method according to any of claims 1 to 8, **characterized in that** for forming the hydrogel bodies (5), an ionically conducting formation based on solid or liquid gel is implemented.

10. The method according to any of claims 1 to 9, **characterized in that** in the middle between the electrode bodies (6), prepared dividing cuts are made so that the multiple electrodes (7, a, b, c) can be manually divided with the desired number of electrode bodies (6).

11. An electrode system, consisting of several multiple electrodes of one product line, manufactured according to any of the preceding claims 1 to 10, **characterized in that** several multiple electrodes (7, a, b, c) with the same or a different number of electrode bodies (6) are joined together by at least one of their longitudinal or transverse edges, completely or partially in abutment for forming application surfaces (9a, 9b) of varying dimensions and varying geometries, wherein all of the electrode bodies (6) of the application surface (9a, 9b) are arranged in the same modular size (A), with respect to the geometric centerlines (x) thereof.

12. The electrode system according to claim 11, **characterized in that** the sensors of all of the electrode bodies (6) of the application surface (9a, 9b) can be coupled by separated lines to a peripheral device.

## Revendications

1. Procédé de fabrication progressive ou en continu d'électrodes multiples (7, a, b, c) souples, auto-adhésives, à usage unique chez l'homme ou l'animal, composées de plusieurs corps d'électrodes (6) identiques, composés respectivement d'une unité hydromel/capteur, pour la transmission non-invasive de signaux électriques de la peau vers un appareil électronique, ou pour la transmission de signaux électriques vers la peau, dans lequel, pour former les corps d'électrode (6) isolés les uns des autres, on introduit dans un matériau support (1) auto-adhésif souple des logements pour un mélange destiné à former les corps d'hydrogel (5), ou le mélange est appliqué à certains endroits sur le matériau support (1), et sur la face détournée de la peau, un connecteur est monté sur les capteurs (4) conducteurs électriques pour le couplage avec les lignes de connexion de l'appareil électronique, et sur le matériau support (1), amené dans la direction longitudinale sous forme de produit en bande sans fin, des corps d'électrodes (6) de construction symétrique et identique sont formés, dans une dimension modulaire (A) définie, par rapport à l'espacement dans la direction longitudinale entre les lignes médianes (x) géométriques des corps d'électrodes dans la direction de 90° à la direction longitudinale, sur une ou plusieurs rangées (10) disposées en parallèle les unes aux autres, s'étendant dans la direction longitudinale du produit en bande,
**caractérisé en ce que** le produit en bande est rogné sur ses côtés longitudinaux de telle sorte que l'espacement (B) entre le bord longitudinal et les lignes médianes (x) géométriques des corps d'électrode (6) adjacents à ce bord longitudinal mesure la moitié de la dimension modulaire (A), et les électrodes multiples (7, a, b, c) sont découpées ou séparées avec le nombre souhaité de corps d'électrodes (6), dans lequel la ligne de coupe ou de séparation est située respectivement au centre entre les corps d'électrodes (6), de sorte que l'espacement (B) du bord à la ligne médiane (x) géométrique des corps d'électrode (6) adjacents au niveau d'au moins trois bords longitudinaux des électrodes multiples (7, a, b, c) mesure respectivement la moitié de la dimension modulaire (A).

2. Procédé de fabrication progressive ou en continu d'électrodes multiples (7) souples, auto-adhésives, à usage unique chez l'homme ou l'animal, composées de plusieurs corps d'électrodes (6) identiques, composés respectivement d'une unité hydrogel/capteur, pour la transmission non-invasive de signaux électriques de la peau vers un appareil électronique, ou pour la transmission de signaux électriques vers la peau, dans lequel pour former les corps d'électrode (6) isolés les uns des autres on introduit dans un matériau support (1) auto-adhésif souple des logements pour un mélange destiné à former les corps d'hydrogel (5), ou le mélange est appliqué à certains endroits sur le matériau support (1), et sur la face détournée de la peau, un connecteur est monté sur les capteurs (4) conducteurs électriques pour le couplage avec les lignes de connexion de l'appareil électronique, et sur le matériau support (1), amené dans la direction longitudinale sous forme de produit en bande sans fin, des corps d'électrodes (6) de construction symétrique et identique sont formés, dans une dimension modulaire (A) définie, par rapport à l'espacement dans la direction longitudinale entre les lignes médianes (x) géométriques des corps d'électrodes dans la direction de 90° à la direction longitudinale, sur une ou plusieurs rangées (10) disposées en parallèle les unes aux autres, s'étendant dans la direction longitudinale du produit en bande,
**caractérisé en ce que** les rangées de corps d'électrode (10) sont disposées de façon décalée les unes par rapport aux autres, de telle sorte que respectivement les lignes médianes (x) géométriques de trois corps d'électrode (6) adjacents forment un triangle équilatéral, le produit en bande est rogné au niveau de ses côtés longitudinaux de telle sorte que l'espacement (B) entre le bord longitudinal et les lignes médianes (x) géométriques des corps d'électrode (6) adjacents à ce bord longitudinal mesure $\frac{\sqrt{3}}{4}$ de la dimension modulaire (A), et les électrodes multiples (7) sont découpées ou séparées avec le nombre souhaité de corps d'électrode (6), dans lequel la ligne de coupe ou de séparation est située respectivement au centre entre les corps d'électrode (6) de sorte que l'espacement (B) du bord à la ligne médiane (x) géométrique des corps d'électrode (6) adjacents au niveau d'au moins trois bords longitudinaux des électrodes multiples (7) mesure respectivement $\frac{\sqrt{3}}{4}$ de la dimension modulaire (A).

3. Procédé selon l'une quelconque des revendications 1 ou 2, **caractérisé en ce que** la dimension de la dimension modulaire A se situe dans une plage de 9 à 120 mm en fonction de la dimension des corps d'hydrogel (5).

4. Procédé selon l'une quelconque des revendications 1 à 3, **caractérisé en ce que** l'espace sans gel entre les corps d'hydrogel (5) individuels mesure au moins 1,5 mm en fonction de la dimension des corps de gel.

5. Procédé selon l'une quelconque des revendications 1 à 4, **caractérisé en ce que** les corps d'hydrogel (5) possèdent un diamètre ou une longueur de bord de 7 à 25 mm.

6. Procédé selon l'une quelconque des revendications 1 à 5, **caractérisé en ce que** les électrodes multiples (7, a, b, c) sont découpées de telle sorte qu'elles présentent au moins deux bords longitudinaux s'étendant de façon rectiligne et en parallèle.

7. Procédé selon l'une quelconque des revendications 1 à 6, **caractérisé en ce que** sur l'un des petits côtés des électrodes multiples (7, a, b, c) une patte de préhension (8) en saillie est disposée pour retirer le revêtement côté peau.

8. Procédé selon l'une quelconque des revendications 1 à 7, **caractérisé en ce que** sur au moins l'un des bords longitudinaux des électrodes multiples (7, a, b, c), le jeu (A/2, B) entre deux corps d'électrode (6) adjacents est identifié par un marquage optiquement perceptible sur la face détournée de la peau.

9. Procédé selon l'une quelconque des revendications 1 à 8, **caractérisé en ce que** pour former les corps d'hydrogel (5), une structure ioniquement conductrice à base de gel solide ou liquide est mise en oeuvre.

10. Procédé selon l'une quelconque des revendications 1 à 9, **caractérisé en ce qu'**au centre entre les corps d'électrode (6), des coupes de séparation préparées sont aménagées de sorte que les électrodes multiples (7, a, b, c) peuvent être séparées manuellement avec le nombre souhaité de corps d'électrode (6).

11. Système d'électrodes, composé de plusieurs électrodes multiples d'une série de fabrication, fabriquées selon l'une quelconque des revendications précédentes 1 à 10, **caractérisé en ce que** plusieurs électrodes multiples (7, a, b, c) avec un nombre identique ou différent de corps d'électrode (6) sont par au moins l'un de leurs bords longitudinaux ou transversaux mis bout à bout complètement ou partiellement pour former des surfaces d'application (9a, 9b) de différentes dimensions et de différentes géométries, dans lequel tous les corps d'électrode (6) de la surface d'application (9a, 9b) sont disposés dans la même dimension modulaire (A), par rapport à leurs lignes médianes (x) géométriques.

12. Système d'électrodes selon la revendication 11, **caractérisé en ce que** les capteurs de tous les corps d'électrode (6) de la surface d'application (9a, 9b) peuvent être couplés par des lignes séparées à un appareil périphérique.

Figur 1

Figur 2

Figur 3

Figur 4

Figur 5

Figur 6

Figur 7

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4336300 A1
- WO 9802088 A
- DE 60114031 T2